(19) Europäisches Patentamt
European Patent Office
Office européen des brevets

(11) **EP 1 653 989 B1**

(12) **FASCICULE DE BREVET EUROPEEN**

(45) Date de publication et mention de la délivrance du brevet:
**15.07.2009 Bulletin 2009/29**

(21) Numéro de dépôt: **04786310.5**

(22) Date de dépôt: **13.08.2004**

(51) Int Cl.:
*A61K 38/10* (2006.01)　　*A61K 38/03* (2006.01)
*A61K 38/16* (2006.01)　　*A61P 31/04* (2006.01)
*A61K 31/7048* (2006.01)

(86) Numéro de dépôt international:
**PCT/FR2004/002142**

(87) Numéro de publication internationale:
**WO 2005/018650 (03.03.2005 Gazette 2005/09)**

(54) **COMPOSITION ANTI-BACTERIENNE PLUS PARTICULIEREMENT CONTRE LES BACTERIES GRAM NEGATIF COMPRENENT UN PEPTIDE ET UN AGENT ANTI-BACTERIEN AVANTAGEUSEMENT HYDROPHOBE**

ANTIBAKTERIELLE ZUSAMMENSETZUNG, VOR ALLEM ZUR BEKÄMPFUNG GRAMNEGATIVER BAKTERIEN, UMFASSEND EIN PEPTID UND EIN VORTEILHAFTERWEISE HYDROPHOBES ANTIBAKTERIELLES AGENS

ANTI-BACTERIAL COMPOSITION, ESPECIALLY FOR CONTROLLING GRAM-NEGATIVE BACTERIA, COMPRISING A PEPTIDE AND AN ADVANTAGEOUSLY HYDROPHOBIC ANTI-BACTERIAL AGENT

(84) Etats contractants désignés:
**AT BE BG CH CY CZ DE DK EE ES FI FR GB GR HU IE IT LI LU MC NL PL PT RO SE SI SK TR**

(30) Priorité: **14.08.2003　FR 0309962**
**14.08.2003　EP 03292030**

(43) Date de publication de la demande:
**10.05.2006　Bulletin 2006/19**

(73) Titulaire: **Diatos**
**75014 Paris (FR)**

(72) Inventeur: **ARRANZ, Valérie**
**F-77122 Monthyon (FR)**

(74) Mandataire: **Breese, Pierre**
**NOVAGRAAF IP**
**3, avenue de l'Opéra**
**75001 Paris (FR)**

(56) Documents cités:
WO-A-01/64738　　　　WO-A-90/12587
WO-A-96/38163　　　　WO-A-98/40401
US-A- 4 526 888

• DARVEAU R P ET AL: "BETA-LACTAM ANTIBIOTICS POTENTIATE MAGAININ 2 ANTIMICROBIAL ACTIVITY IN VITRO AND IN VIVO" ANTIMICROBIAL AGENTS AND CHEMOTHERAPY, AMERICAN SOCIETY FOR MICROBIOLOGY, WASHINGTON, DC, US, vol. 35, no. 6, juin 1991 (1991-06), pages 1153-1159, XP008010120 ISSN: 0066-4804

**Description**

La présente invention concerne le domaine des traitements anti-bactériens et plus particulièrement des compositions pour traiter les infections par des bactéries gram négatif chez l'homme, l'animal ou les plantes.

Il a été décrit dans l'art antérieur des peptides capables de détruire les bactéries (Park CB, Kim HS, Kim SC. Biochem Biophys Res Commun. 1998 Mar 6;244(1):253-7). Il a également été rapporté dans la demande de brevet internationale PCT No. WO 01/64738 des peptides capables de réagir avec les aminoglycanes et de transporter dans des cellules eucaryotes ou procaryotes des molécules d'intérêt.

La quantité de molécules antibiotiques pénétrant dans la bactérie dépend de sa structure et de mécanismes impliqués dans le transport de substrats. Les bactéries Gram négatif se distinguent structurellement des bactéries Gram positif par la présence de deux membranes constituant l'enveloppe bactérienne. Si toutes les bactéries ont une membrane interne, les bactéries Gram négatif ont une membrane externe unique supplémentaire. Cette membrane externe hydrophobe constitue une barrière semi-perméable qui s'oppose à la pénétration des antibiotiques mais des porines, protéines formant des canaux, permettent l'entrée de petits solutés hydrophiles comme les éléments nutritifs et les antibiotiques de la famille des pénicillines et tétracyclines, mais excluent la pénétration de grosses molécules hydrophiles et les antibiotiques de la famille des macrolides/kétolides.

Une cause importante des échecs thérapeutiques contre les bactéries Gram négatif est l'émergence de souches résistantes. Certaines résistances sont liées à une réduction de la perméabilité des membranes bactériennes (modifications quantitatives/qualitatives de porines). D'autres résistances tiennent à la présence d'une protéine membranaire qui provoque le rejet de l'antibiotique par un mécanisme d'efflux actif. Le développement de nouveaux types de molécules anti-bactériennes ou l'utilisation d'antibiotiques commerciaux non actifs sur les bactéries Gram négatif requiert leur entrée et leur délivrance au travers de membranes bactériennes sélectives.

La présente invention a précisément pour but d'offrir de nouvelles compositions permettant de traiter efficacement les infections par les bactéries Gram négatif même lorsque celles-ci ont développé une résistance aux antibiotiques.

Ce but est atteint grâce à l'utilisation de peptides capables de traverser la membrane externe des bactéries Gram négatif et, via cette translocation de la membrane, de délivrer des molécules d'intérêt qui ne peuvent pas sinon pénétrer à l'intérieur des bactéries, du fait de leurs propriétés physico-chimiques.

On entend par pénétration à l'intérieur des bactéries, le fait que les peptides de l'invention facilitent ou permettent la pénétration des molécules d'intérêt dans les bactéries. Les termes pénétration et internalisation seront utilisés comme synonymes ci-après.

Les travaux réalisés dans le cadre de la présente invention ont concerné le Bodipy et la Tétramethylrhodamine qui sont des molécules fluorescentes hydrophobes exclues par la membrane externe des bactéries Gram négatif. Ces traceurs fluorescents ont été choisis afin d'évaluer les propriétés d'internalisation des peptides de l'invention liés chimiquement à des molécules hydrophobes. La translocation des traceurs fluorescents dans la bactérie Gram négatif a été évaluée qualitativement sur *Escherichia coli* et *Pseudomonas aeruginosa.*

La présente invention a donc tout d'abord pour objet une composition anti-bactérienne, plus particulièrement dirigée contre les bactéries Gram négatif, comprenant l'association :

    a) d'au moins un peptide de 10 à 25 résidus d'acide aminé comprenant :

        i) deux domaines chargés positivement à pH neutre constitués chacun de 3 à 9 résidus d'acide aminé dont les deux tiers au moins sont des acides aminés cationiques,
        ii) entre lesdits domaines chargés positivement, un groupe de deux à trois résidus d'acide aminé non cationique,
        iii) à l'une et ou l'autre des extrémités N ou C terminales du peptide, un groupe de 0 à 10 et de préférence de 0 à 5 résidus d'acide aminé choisis dans le groupe comprenant des acides aminés non hydrophobes et des acides aminés chargés positivement, mais dans le cas d'un résidu d'acide aminé chargé positivement celui-ci n'est pas directement adjacent aux domaines chargés positivement.

    b) d'au moins un composé anti-bactérien.

Ainsi, les peptides de l'invention sont tout particulièrement utiles pour la préparation d'une composition pharmaceutique destinée au traitement d'une infection, plus particulièrement par des bactéries gram-négatif, composition dans laquelle ledit peptide traverse la membrane des bactéries de façon à délivrer à l'intérieur de celles-ci un composé antibactérien auquel il est associé dans ladite composition.

Avantageusement, dans les peptides de l'invention ci-dessus, les acides aminés cationiques des deux domaines chargés positivement sont choisis dans le groupe comprenant l'arginine et la lysine.

On préfère dans les peptides de l'invention ci-dessus que les acides aminés non cationiques du groupe entre lesdits domaines chargés positivement soient des acides aminés :

- non hydrophobes, choisis par exemple dans le groupe comprenant : l'acide glutamique, la serine, la glycine, et la glutamine, ou
- la leucine (acide aminé hydrophobe).

L'orientation des séquences d'acide aminé selon l'invention est typiquement de N-terminale vers C-terminale. Cependant, selon un autre mode de réalisation, l'orientation peut être inversée, c'est-à-dire que les séquences d'acide aminés sont orientées de C-terminal vers N-terminal.

Des peptides préférés pour les compositions selon l'invention sont choisis dans le groupe comprenant les séquences suivantes (orientation N-terminal vers C-terminal) :

- DPV3 : Arg Lys Lys Arg Arg Arg Glu Ser Arg Lys Lys Arg Arg Arg Glu Ser (SEQ ID NO.1)
- DPV3.10 : Arg Lys Lys Arg Arg Arg Glu Ser Arg Arg Ala Arg Arg Ser Pro Arg His Leu (SEQ ID NO.2)
- DPV6 : Gly Arg Pro Arg Glu Ser Gly Lys Lys Arg Lys Arg Lys Arg Leu Lys Pro (SEQ ID NO.3)
- DPV7 : Gly Lys Arg Lys Lys Lys Gly Lys Leu Gly Lys Lys Arg Asp Pro (SEQ ID NO.4)
- DPV7b : Gly Lys Arg Lys Lys Lys Gly Lys Leu Gly Lys Lys Arg Pro Arg Ser Arg (SEQ ID NO.5)
- DPV15 : Leu Arg Arg Glu Arg Gln Ser Arg Leu Arg Arg Glu Arg Gln Ser Arg (SEQ ID NO.6)
- DPV15b : Gly Ala Tyr Asp Leu Arg Arg Arg Glu Arg Gln Ser Arg Leu Arg Arg Arg Glu Arg Gln Ser Arg (SEQ ID NO.7)
- DPV1047 : Val Lys Arg Gly Leu Lys Leu Arg His Val Arg Pro Arg Val Thr Arg Met Asp Val (SEQ ID NO.8)
- DPV11 : Ala Lys Thr Gly Lys Arg Lys Arg Ser Gly (SEQ ID NO.9)
- DPV1121 : Val Lys Arg Gly Leu Lys Leu Arg Gln Lys Tyr Asn Lys Arg Ala Met Asp Tyr (SEQ ID NO.11)

Parmi ceux-ci, l'invention concerne tout spécialement les peptides suivants : DPV3, DPV3.10, DPV6, DPV7, DPV7b, DPV15 et DPV15b.

L'alignement des séquences ci-dessus met en évidence des domaines chargés positivement de séquences suivantes :

- Arg Lys Lys Arg Arg Arg (SEQ ID NO.13)
- Arg Pro Arg (SEQ ID NO.14)
- Lys Arg Lys Lys Lys Gly Lys (SEQ ID NO.15)
- Arg Arg Glu Arg (SEQ ID NO.16)
- Arg Arg Arg Glu Arg (SEQ ID NO.17)
- Arg Arg Ala Arg Arg Ser Pro Arg (SEQ ID NO.18)
- Lys Lys Arg Lys Arg Lys Arg Leu Lys (SEQ ID NO.19)
- Lys Lys Arg (SEQ ID NO.20)
- Lys Lys Arg Pro Arg Ser Arg (SEQ ID NO.21)
- Arg Leu Arg Arg Glu Arg (SEQ ID NO.22)
- Arg Leu Arg Arg Arg Glu Arg (SEQ ID NO.23)

De préférence, les domaines de séquences SEQ ID NO. 13 à 17 sont du côté de l'extrémité N terminale du peptide, alors que les domaines de séquences SEQ ID NO. 18 à 23 sont du côté de l'extrémité C terminale du peptide.

L'alignement des séquences ci-dessus a aussi mis en évidence des groupes de deux à trois résidus d'acide aminé non cationique entre les domaines chargés positivement de séquences suivantes :

Glu Ser, Glu Ser Gly, Leu Gly, Gln Ser

Les composés anti-bactériens présents dans les compositions selon l'invention sont de préférence choisis parmi ceux présentant des propriétés physico-chimiques les rendant incapables de traverser la membrane des bactéries Gram négatif. Tout préférentiellement, il s'agit de composés anti-bactériens hydrophobes. A titre d'exemples de tels composés, on peut citer les antibiotiques de la famille de macrolides, des ketolides comme l'erythromycine, la clarithromycine, l'azithromycine, la télithromycine.

Les composés anti-bactériens peuvent être aussi des oligonucléotides antisens.

L'évaluation des peptides DPV ci-dessus à montrer leur capacité à traverser les membranes des bactéries Gram négatif *E. coli* ou *P. aeruginosa* et de délivrer le Bodipy dans la bactérie, alors que celui-ci est une molécule hydrophobe normalement exclue par la membrane externe des bactéries Gram négatif qui représente une barrière semi-perméable. De plus, il apparaît que ces peptides ont la capacité de traverser la membrane externe des deux souches de bactéries Gram négatif et de s'accumuler dans le cytoplasme bactérien par un mécanisme non énergie-dépendant et non toxique pour la bactérie.

Les travaux réalisés dans le cadre de l'invention, ont mis en évidence quelques différences d'internalisation entre les deux souches de bactéries *P. aeruginosa* et *E. coli.* Ainsi il semble que le peptide DPV7b soit plus internalisants dans P. *aeruginosa* que dans *E. coli.* Cette différence pourrait s'expliquer par des différences de structures de la membrane externe entre les deux souches de bactéries.

Ces peptides sont donc utiles pour la préparation de compositions pharmaceutiques anti-bactériennes, plus particulièrement contre les bactéries gram négatif, où ils sont associés à un ou plusieurs agents anti-bactériens.

Les compositions de l'invention sont utiles tant à titre préventif que curatif.

Les compositions selon l'invention comprennent également avantageusement un ou plusieurs véhicules, diluants ou excipients pharmaceutiquement acceptables généralement utilisés avec ce type d'agents.

Les peptides de l'invention peuvent être préparés par synthèse chimique ou par génie génétique dans une cellule procaryote comme une bactérie, dans une cellule eucaryote comme une levure, une cellule CHO (Chinese Hamster Ovary), une cellule NSO (Mouse myeloma cells), chez un animal transgénique, par exemple dans le lait de lapin, de chèvre, de brebis, de vache, etc... transgéniques ou dans une plante transgénique comme, par exemple, dans des plants de tabac, etc....

L'invention concerne aussi des équivalents fonctionnels des peptides définis ci-dessus, tels que des peptides comprenant des modifications issues de processus post-traductionnels comme la glycosylation ou de modifications chimiques telles que le couplage avec des lipides, sucres, séquences de nucléotides dès lors que ces modifications ne modifient pas l'activité anti-bactérienne et/ou antifongique desdits peptides conformément aux tests donnés dans la partie expérimentale ci-après. Les équivalents fonctionnels comprennent aussi des peptides dont un ou plusieurs acides aminés sont des acides aminés de conformation D. L'invention couvre également les rétro-peptides et les rétro-inverso-peptides.

L'association des compositions selon l'invention peut être constituée d'un ou plusieurs peptides décrits ci-dessus et d'un ou plusieurs composés anti-bactériens, aussi sauf indication contraire, le singulier employé pour la définition des agents actifs (peptide et composé anti-bactérien) de la composition couvre le pluriel.

La composition selon l'invention peut être réalisée par l'association de peptide(s) et de composé(s) anti-bactérien(s) en mélange ou d'un produit dans lequel un ou plusieurs peptides identiques ou différents sont liées par covalence à un ou plusieurs composés identiques ou différents, éventuellement par l'intermédiaire d'un bras espaceur. De tels produits sont notamment des produits de formule (I) qui seront décrits ci-après.

Dans le cas de l'administration de peptide et de composé anti-bactérien en mélange, ces deux agents actifs de la composition anti-bactérienne de l'invention peuvent être présentés de façon séparée, chacun sous une forme pharmaceutique appropriée, et réunis dans un même emballage. Toutefois, pour faciliter l'administration simultanée des agents actifs, on préfère généralement préparer le médicament sous une seule forme pharmaceutique contenant les deux ingrédients actifs en mélange, ainsi éventuellement qu'un excipient pharmaceutique approprié.

Bien entendu, un produit constitué d'un peptide lié directement ou indirectement à un composé anti-bactérien doit être considéré comme constituant à lui seul une association selon l'invention et qui peut être utilisé comme ingrédient actif unique.

Par exemple, un peptide et un composé anti-bactérien peuvent être combinés en établissant une liaison chimique entre ceux-ci. On peut notamment amidifier une fonction amine du peptide, ou estérifier une ou plusieurs fonctions alcool du peptide, avec un groupement acide présent au niveau d'un composé anti-bactérien ou dérivé de celui-ci. On obtient ainsi un produit d'amidification qui constitue le produit actif de la composition de l'invention. On peut également ajouter à l'une et/ou l'autre des extrémités N et/ou C terminal du peptide un résidu d'acide aminé dont la chaîne latérale permet le couplage avec un composé anti-bactérien, comme par exemple un résidu de cystéine dont le groupe SH est réactif. Des exemples de tels produits sont ceux de formule (I) qui sont décrits ci-dessous.

En effet, l'invention a également pour objet de nouveaux produits où le peptide et le composé anti-bactérien sont liés l'un à l'autre par covalence, éventuellement par l'intermédiaire d'au moins un bras espaceur.

De tels produits sont notamment ceux qui répondent à la formule (I) suivante :

$$(A\text{-})_m(X)_p(\text{-}P)_n \qquad (I)$$

dans laquelle : A est le reste d'un composé anti-bactérien, P est le reste d'une peptide, tels que définis précédemment, et X représente soit une liaison covalente entre A et P, soit un bras espaceur reliant au moins un reste A à au moins un reste P, m est un nombre entier pouvant aller de 1 à 3, n est un nombre entier pouvant aller de 1 à 3, et p représente zéro ou un nombre entier au plus égal au plus grand des nombres m et n.

On peut en effet, soit greffer un ou plusieurs restes A et/ou P sur un seul bras espaceur, soit greffer un ou plusieurs groupes A-X sur un reste P (et alors m = p et n = 1), soit greffer un ou plusieurs groupes X-P sur un reste A (et alors n = p et m = 1). Lorsque p = zéro, soit un ou plusieurs restes A sont liés directement à un reste P (et n = 1), soit un ou plusieurs restes P sont liés directement à un reste A (et m = 1).

Les produits de formule (I) peuvent être utilisés sous forme de sels, en particulier sous forme de sels de métaux alcalins tels que des sels de sodium ou de potassium ; ces sels sont par exemple ceux des groupements phosphates, s'ils sont présents, des groupements phénoliques (cas de l'acide salicylique), etc. On peut également utiliser les produits de formule (I), le cas échéant, sous forme de sels d'addition (par exemple sous forme de chlorhydrate) lorsque ces produits contiennent un groupement amine.

Les liaisons entre le bras espaceur et les restes A et P ou directement entre A et P sont des liaisons covalentes. Ces liaisons covalentes peuvent être formées, comme indiqué précédemment, entre des groupes ester carboxylique, amide carboxylique, ester thiocarboxylique ou amide thiocarboxylique.

Les restes de composé anti-bactérien (A) et de peptide (P) sont des dérivés de composé anti-bactérien ou de peptide, dont un ou plusieurs groupes chimiques ont été soit supprimés soit modifiés de façon à permettre la formation de liaison covalentes directement entre A et P ou indirectement par l'intermédiaire d'un bras espaceur.

Il peut s'agir de fonctions acyles de composés anti-bactériens possédant un groupe carboxylique capable de former une liaison avec le bras espaceur ou le peptide, ce dernier possédant une amine primaire ou un groupe hydroxyle apte à former une liaison covalente avec le bras espaceur ou le composé anti-bactérien.

Les bras espaceurs peuvent être notamment des restes bivalents de composés aliphatiques bi-fonctionnels, comme des composés ayant à chacune de leurs extrémités des groupes fonctionnels réactifs permettant chacun de former des liaisons covalentes avec A et avec P. Ces composés peuvent être par exemple des composés qui possèdent à la fois un groupe amino et un groupe carboxylique (ou thiocarboxylique), ou encore des composés qui possèdent à la fois un groupe amino et un groupe hydroxyle.

Dans la formule (I), le groupe X (en faisant abstraction de ses groupes fonctionnels d'extrémité) représente notamment un groupe aliphatique divalent éventuellement interrompu par un ou plusieurs hétéroatomes - O - ou - S - ou par un ou plusieurs groupements hétéroatomiques - NH - ou - CO - NH -.

Les composés capables de donner, après réaction avec le peptide et le composé anti-bactérien ou leurs dérivés, des produits de formule (I) dans lesquels A et P sont reliés par des bras espaceurs, sont par exemple des acides alpha-, bêta- ou gamma-amino alcanecarboxyliques, en particulier des acides alpha - aminés naturels tels que la glycine, l'alanine, la valine ou la leucine, ou encore des peptides, notamment des dipeptides ou des tripeptides. Comme indiqué dans les exemples, il peut s'agir avantageusement d'un résidu de cystéine.

Les agents espaceurs peuvent également être des acides hydroxy-carboxyliques tels que les acides lactique, glycolique, les acides aldoniques (gluconique, mannonique, galactonique, ribonique, arabinonique, xylonique et érythronique) et les lactones ou dilactones correspondantes (par exemple lactide, glycolide, delta-glucolonactone, delta-valéronactone), ou encore les acides aldariques.

Les groupes fonctionnels éventuellement présents sur le bras espaceur et non impliqués dans la liaison avec A ou P peuvent être utilisés pour greffer d'autres restes A et/ou P de façon à obtenir des composés de formule (I) pour lesquels m et/ou n sont supérieurs à 1. C'est le cas par exemple des groupes hydroxyles des hydroxyacides, du second groupe carboxylique des acides aminés diacides carboxyliques, du second groupe amino des aminoacides diaminés, du groupe hydroxyle des acides aminés hydroxylés, etc.

Le bras espaceur peut avantageusement constitué une molécule de liaison apte à permettre la libération retardée de l'un et/ou l'autre des restes A ou P, notamment en les protégeant d'une dégradation après administration. Le bras espaceur peut aussi constitué une molécule de vectorisation permettant de cibler un organe ou tissu particulier pour la délivrance des restes de composé anti-bactérien.

Pour préparer les composés de formule (I), on utilise les méthodes classiques de la synthèse organique. Par exemple, pour préparer des amides ou des esters, on peut faire réagir un composé carboxylique sous la forme d'un halogénure d'acide carboxylique (ou thiocarboxylique), ou sous la forme d'un anhydride mixte, ou sous la forme d'un ester activé, par exemple un ester de p-nitrophényle. On peut également activer l'acide à l'aide d'un agent de couplage tel que le dicyclohexylcarbodiimide.

Comme les composés de formule (I) comprennent des restes de peptide, on peut les préparer en utilisant en particulier les méthodes connues dans la chimie des peptides.

Bien entendu, lorsque les composés dont dérivent A, P ou X de la formule (I), comprennent plusieurs fonctions susceptibles de réagir, il convient d'opérer soit en utilisant les réactifs en proportions stoechiométriques (selon le nombre de produits précurseurs de A et/ou de P que l'on veut faire réagir), soit en protégeant temporairement les fonctions réactives dont on ne souhaite pas qu'elles réagissent. On utilise pour cela les méthodes de protection temporaire desdites fonctions réactives. Ces méthodes de protection temporaire sont bien connues, notamment celles qui ont été développées lors des recherches concernant la synthèse des peptides. Par exemple, les groupements -NH$_2$ peuvent être protégés par des groupements carbobenzoxy, phtaloyle, t-butoxycarbonyle, trifluoroacétyle, toluènesulfonyle ; les groupements carboxyliques peuvent être protégés sous la forme d'esters benzyliques, d'esters de tétrahydropyranyle ou d'esters de t-butyle ; les alcools peuvent être protégés sous la forme d'esters (par exemple acétates), sous la forme d'éthers de tétrahydropyranyle, d'éthers benzyliques ou d'éthers de trityle, ou encore sous la forme d'acétals (y compris sous la forme d'acétonides dans le cas des glycols vicinaux). Les réactions de protection et de déprotection éventuelle de divers groupes chimiques sont connues et décrites dans la littérature.

Les réactions de phosphatation ou de déphosphatation de l'alcool primaire des nucléotides ou nucléosides peuvent être mises en oeuvre en utilisant les enzymes naturelles (par exemple phosphatases, phosphokinases).

Les compositions anti-bactériennes de l'invention, et notamment celles comprenant un composé de formule (I), peuvent être administrées par l'un quelconque des modes d'administration acceptés pour les agents thérapeutiques et bien entendu par voie orale, sublinguale, nasale, pulmonaire, rectale ou parentérale (par exemple intravasculaire, intramusculaire, transcutanée, intra-articulaire). On peut aussi citer l'administration systémique, topique ou encore centrale, par exemple par voie chirurgicale intracrânienne ou encore l'administration intra-oculaire. On peut citer aussi l'implantation sous-cutanée d'implants biodégradable.

A cet effet, elles peuvent être présentées sous toute forme permettant l'administration par :

- voie orale (en particulier sous la forme de gélules, de solutions ou émulsions buvables, de poudres, de gels, de granulés, de tablettes ou de comprimés), de comprimés, gélules, capsules molles, y compris les formulations à libération différée ou prolongée, pilules, poudres, granules, élixirs, teintures, suspensions, sirops et émulsions. Cette forme de présentation est plus particulièrement adaptée au passage de la barrière intestinale et à l'utilisation la plus commune des composés anti-bactériens et/ou antifongiques.
- voie parentérale, généralement par injection intramusculaire ou intraveineuse par perfusion. Les compositions injectables peuvent être préparées sous des formes classiques, soit en suspension ou solution liquide soit sous forme solide convenant à une dissolution extemporanée dans un liquide adéquat, y compris les formulations à libération différée ou prolongée comme l'inclusion des peptides dans des micro ou nano particules biodégradables de formulation lipidique ou de formulation de dextran ou encore de PLGA ou équivalents. Cette forme de présentation est plus particulièrement adaptée au passage de la barrière hémato-encéphalique et à l'utilisation hospitalière des composés anti-bactériens et/ou antifongiques.

Une possibilité pour l'administration parentérale utilise l'implantation d'un système à libération lente ou libération prolongée qui assure le maintien d'un niveau constant de dose.

Une autre possibilité consiste à fixer par adsorption ou autre les peptides de l'invention sur un support, comme un cathéter, une prothèse ou une colle biologique.

- voie nasale (par exemple des solutions à administrer sous forme de gouttes ou de pulvérisations),
- voie pulmonaire (solutions en flacon pressurisé pour aérosols),
- voie rectale (suppositoires),
- voie cutanée (par exemple crèmes, onguents ou dispositifs transdermiques, encore appelés timbres ou patches),
- voie transmuqueuse comme par exemple par voie sublinguale (solutions en flacon pressurisé, ou comprimés à délitement buccal).

Ces formes pharmaceutiques sont préparées de façon usuelle et peuvent contenir des excipients et véhicules classiques appropriés.

D'autres préparations topiques habituelles comprennent les crèmes, les onguents, les lotions, les gels et les sprays aérosols. Ces derniers sont plus particulièrement adaptés pour le traitement des infections broncho-pulmonaires bactériennes et/ou fongiques.

Les compositions de l'invention peuvent aussi être utilisées dans le domaine cosmétique, à titre essentiellement préventif, et consistant alors en crèmes, vernis à ongle, produits d'hygiène des organes génitaux, pâtes de dentifrices, solutions d'hygiène buccales ou à inclure dans des micro-particules à diffusion lente, en phase aqueuse, incluses par exemple dans des couches-culottes, des cotons-tiges, des pansements, des cotons à démaquiller, des serviettes hygiéniques ou encore des litières pour animaux.

En fonction du mode d'administration, les composés peuvent être sous forme solide, semi-solide ou liquide.

Pour les compositions solides, tels que comprimés, pilules, poudres ou granulés à l'état libre ou inclus dans des gélules, l'association peut être combinée avec :

- des diluants, par exemple le lactose, le dextrose, le sucrose, le mannitol, le sorbitol, la cellulose et/ou la glycine ;
- des lubrifiants, par exemple la silice, le talc, l'acide stéarique, son sel de magnésium ou de calcium et/ou le polyéthylèneglycol ;
- des liants, par exemple le silicate de magnésium et d'aluminium, la pâte d'amidon, la gélatine, la gomme adragante, la méthylcellulose, la carboxyméthylcellulose sodique et/ou la polyvinylpyrrolidone ; le cas échéant,
- des désintégrants, par exemple l'amidon, l'agar, l'acide alginique ou son sel de sodium, ou des mélanges effervescents ; et/ou
- des absorbants, colorants, aromatisants et édulcorants. Les excipients peuvent être par exemple du mannitol, lactose, amidon, stéarate de magnésium, saccharine sodique, talc, cellulose, glucose, sucrose, carbonate de magnésium et analogues de qualité pharmaceutique.

Pour les compositions semi-solides, telles que suppositoires, l'excipient peut, par exemple, être une émulsion ou suspension grasse, ou à base de polyalkylèneglycol, tel que le polypropylène-glycol.

Les compositions liquides, en particulier injectables ou à inclure dans une capsule molle, peuvent être préparées par exemple par dissolution, dispersion, etc. du principe actif dans un solvant pharmaceutiquement pur tel que, par exemple, l'eau, le sérum physiologique, le dextrose aqueux, le glycérol, l'éthanol, une huile et analogues.

Les compositions selon l'invention peuvent également être administrés sous la forme de systèmes de libération du type liposomes, tels que sous la forme de petites vésicules unilamellaires, de grandes vésicules unilamellaires et de vésicules multilamellaires. Les liposomes peuvent être formés à partir d'une diversité de phospholipides, contenant du cholestérol, de la stéarylamine ou des phosphatidylcholines. Dans une forme d'exécution, un film de composants liquides peut être hydraté avec une solution aqueuse du médicament pour former une couche lipidique encapsulant le médicament.

Les compositions selon l'invention peuvent être stérilisées et/ou contenir des adjuvants et substances auxiliaires non toxiques tels que des agents de conservation, de stabilisation, de mouillage ou d'émulsification, des agents favorisant la dissolution, des sels pour régler la pression osmotique et/ou des tampons. En outre, elles peuvent également contenir d'autres substances présentant un intérêt thérapeutique. Les compositions sont préparées, respectivement, par des procédés classiques de mélange, granulation ou enrobage et elles contiennent d'environ 0,1 à 75%, de préférence d'environ 1 à 50 %, de principe actif.

Les peptides et agents anti-bactériens de l'association de la composition selon l'invention peuvent être également couplés avec des polymères solubles tels que des supports de médicament ciblables. De tels polymères peuvent comprendre la polyvinylpyrrolidone, le copolymère pyrane, le polyhydroxypropyl-méthacrylamide-phénol, le polyhydroxy-éthyl-aspanamide-phénol ou le poly(oxyde d'éthylène)-polylysine substitué par des résidus palmitoyle, le dextran. En outre, les composés selon la présente invention peuvent être couplés à une classe de polymères biodégradables utiles pour réaliser une libération maîtrisée d'un médicament, par exemple, le poly(acide lactique), la poly(epsilon-caprolactone), le poly(acide hydroxybutyrique), les polyorthoesters, les polyacétals, les polydihydropyranes, les polycyanoacrylates et les copolymères d'hydrogel séquencés réticulés ou amphipatiques.

La posologie pour l'administration des compositions selon l'invention est choisie en fonction de nombreux facteurs y compris le type, l'espèce, l'âge, le poids, le sexe et l'état médical du sujet, la gravité de l'état à traiter, la voie d'administration ; l'état des fonctions rénale et hépatique du sujet et la nature du composé particulier, ou sel, employé. Un médecin ou vétérinaire normalement expérimenté déterminera facilement, et prescrira, la quantité efficace pour prévenir, contrarier ou stopper le progrès de l'état médical à traiter.

Une composition selon l'invention peut contenir de 0,1 à 99%, de préférence 1 à 70% de principe actif.

A titre d'exemples, les posologies orales des compositions selon l'invention seront comprises entre environ 0,5 et 1 mg/jour par voie orale et, de préférence fournies sous la forme de comprimés contenant 0,5, 1, 2,5, 5, 10, 15, 25, 50, 100, 250, 500 et 1.000 mg de principe actif. Les concentrations plasmatiques efficaces seront obtenues à partir d'une posologie allant de 0,002 mg à 50 mg par kg de poids corporel et par jour.

Les compositions de l'invention peuvent être administrés sous la forme de doses quotidiennes uniques, ou en deux, trois ou quatre doses par jour.

D'autres avantages et caractéristiques de l'invention apparaîtront des exemples qui suivent, donnés à titre illustratif, et dans lesquels il sera fait référence aux dessins en annexe où :

La figure 1 représente la formule du Bodipy® FL N-(2-aminoethyl) maléimide.
La figure 2 représente la formule du Tetramethylrhodamine-6-maléimide.
La figure 3 représente l'internalisation des conjugués DPV~Bodipy dans *E. coli*.
La figure 4 représente l'immuno-marquage de la membrane externe après internalisation du conjugué DPV3-bodipy.
La figure 5 représente l'internalisation des conjugués DPV~Bodipy dans P.*aeruginosa.*
La figure 6 donne des images de microscopie confocal de *P. aeruginosa.*
La figure 7 représente l'internalisation du conjugué DPV3~TMR dans *E. coli.*

I - Matériels et Méthodes.

I.1) Traceurs fluorescents.

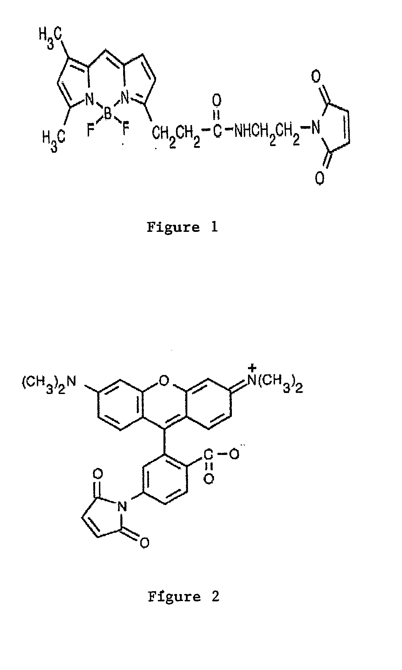

- Le Bodipy® FL*N*-(2-aminoethyl) maleimide (Bodipy) (Molecular Probes Cat# B-10250), dont la formule moléculaire est $C_{20}H_{21}BF_2N_4O_3$, Le poids moléculaire est de 414,22 Da, l'absorbance de 504nm et l'émission de 510nm (fluorescence verte), et la formule développée est donnée à la figure 1.
- Tetramethylrhodamine-6-maleimide (TMR) (Molecular Probes Cat# T-6028), dont la formule moléculaire est

$C_{28}H_{23}N_3O_5$, le poids moléculaire est de 481,51 Da, l'absorbance de 541nm et l'émission 567nm (fluorescence rouge) et la formule développée est donnée à la figure 2.

Ces deux molécules fluorescentes contiennent un groupe réactif maléimide permettant le couplage chimique sur la fonction thiol de la cystéine du peptide.

I.2) Les vecteurs peptidiques (DPVs).

Les peptides de séquences ci-dessous ont été utilisés :

- DPV3 : Arg Lys Lys Arg Arg Arg Glu Ser Arg Lys Lys Arg Arg Arg Glu Ser (SEQ ID NO.1) avec un résidu Cys (Cystéine) à son extrémité C terminale,
- DPV3.10 : Arg Lys Lys Arg Arg Arg Glu Ser Arg Arg Ala Arg Arg Ser Pro Arg His Leu (SEQ ID NO.2) avec un résidu Cys à son extrémité C terminale,
- DPV6 : Gly Arg Pro Arg Glu Ser Gly Lys Lys Arg Lys Arg Lys Arg Leu Lys Pro (SEQ ID NO.3) avec un résidu Cys à son extrémité C terminale,
- DPV7 : Gly Lys Arg Lys Lys Lys Gly Lys Leu Gly Lys Lys Arg Asp Pro (SEQ ID NO.4) avec un résidu Cys à son extrémité C terminale,
- DPV7b : Gly Lys Arg Lys Lys Lys Gly Lys Leu Gly Lys Lys Arg Pro Arg Ser Arg (SEQ ID NO.5) avec un résidu Cys à son extrémité C terminale,
- DPV15 : Leu Arg Arg Glu Arg Gln Ser Arg Leu Arg Arg Glu Arg Gln Ser Arg (SEQ ID NO.6) avec un résidu Cys à son extrémité C terminale,
- DPV15b : Gly Ala Tyr Asp Leu Arg Arg Arg Glu Arg Gln Ser Arg Leu Arg Arg Arg Glu Arg Gln Ser Arg (SEQ ID NO. 7) avec un résidu Cys à son extrémité N terminale,
- DPV1047 : Val Lys Arg Gly Leu Lys Leu Arg His Val Arg Pro Arg Val Thr Arg Met Asp Val (SEQ ID NO.8) avec un résidu Cys à son extrémité N terminale,
- DPV11 : Ala Lys Thr Gly Lys Arg Lys Arg Ser Gly (SEQ ID NO.9) avec un résidu Cys à son extrémité C terminale,
- DPV12 : Gln Gly Lys Ser Lys Arg Glu Lys Lys Asp Arg Val Phe (SEQ ID NO.10) avec un résidu Cys à son extrémité C terminale,
- DPV1121 : Val Lys Arg Gly Leu Lys Leu Arg Gln Lys Tyr Asn Lys Arg Ala Met Asp Tyr (SEQ ID NO.11) avec un résidu Cys à son extrémité N terminale,
- DPV19 : Asn Pro Gly Val Ser Thr Val Val Leu Gly Ala Tyr Asp Leu Arg Arg Arg Glu Arg Gln Ser Arg (SEQ ID NO. 12) avec un résidu Cys à son extrémité N terminale.

Les synthèses peptidiques ont été réalisées selon les techniques connues de l'homme de métier. Les peptides sont solubles dans l'eau.
Les peptides possèdent un résidu Cystéine en position N- ou C- terminale afin de permettre la conjugaison au traceur fluorescent.

I.3) Les produits contrôles.

Bodipy et TMR sont couplés chimiquement à un résidu cystéine et servent de témoin négatif d'internalisation.

I.4) Méthode de couplage chimique.

Les solutions de Bodipy ou TMR sont préparées à une concentration finale de 50mM en Diméthylformamide (DMF). Les solutions de DPVs sont préparées à une concentration finale de 10mM en DMF. 200 µl de la solution de Bodipy ou TMR sont mélangés à 700 µl de la solution de DPV. Après une incubation de 2 heures à température ambiante, à l'obscurité, 2ml d'H2O et 8ml de dichlorométhane (DCM) sont ajoutés. La solution est mélangée au vortex et centrifugée 2 minutes à 3000g. La phase aqueuse est prélevée et conservée. Quatre extractions successives au DCM sont réalisées. Les phases aqueuses sont rassemblées dans un flacon de verre et placées 1 heure à -80°C avant d'être lyophilisées au minimum 18 heures. La poudre obtenue est stockée sous argon à -20°C à l'abri de la lumière.

I.5) Conservation des conjugués en solution.

Les conjugués DPV~Bodipy et DPV~TMR sont conservés dilués à 3mM dans H2O à -20°C, à l'abri de la lumière.

I.6) <u>Analyse HPLC des conjugués</u>.

- Pour les conjugués Bodipy :

  Colonne Luna 100Å 3μ C18 100x4.6 mm
  Solvant A: 0.1% TFA dans de l' $H_2O$
  Solvant B: 0.1% TFA dans de l'acétonitrile (CAN)
  Gradient: 5% B à 60% en 10 min, 60% B à 90% en 1 min, 90% B pendant 3 min, 5% B pendant 2 min
  Flux: 1.2 ml/min; volume injecté: 10 μl; la concentration de l'échantillon injecté est de 1 mg/ml en 0.1% TFA
  Détecteur: DAD: 214nm, 300 nm.

- Pour les conjugués TMR :

  Colonne Luna 100Å 3μ C18 100x4.6 mm
  Solvant A : 0.1% TFA dans de l'$H_2O$
  Solvant B : 0.1% TFA dans de l'acétonitrile (CAN)
  Gradient : 5% B à 60% en 10 min, 60% B à 90% en 1 min, 90% B pendant 3 min, 5% B pendant 2 min
  Flux : 1.2 ml/min; volume injecté: 20 μl; la concentration de l'échantillon injecté est de 1 mg/ml en 0.1% TFA
  Détecteur: DAD: 220 nm.

I.7) <u>Souches bactériennes</u>.

- *Escherichia coli* ATCC 25922
- *Pseudomonas aeruginosa* ATCC 27853

I.8) <u>Protocole d'internalisation</u>.

I.8.a) <u>Evaluation de la pénétration des conjugués dans la bactérie à 37°C.</u>

Les bactéries en phase exponentielle de culture sont centrifugées et lavées 3 fois avec le tampon phosphate de sodium 10mM, pH7.4 (tampon NAPB). La concentration bactérienne est ajustée à 1x10⁶ ufc/ml (Unités Formant Colonies) dans le tampon NAPB. 50 μl de la suspension bactérienne sont déposés sur lame de poly-L-Lysine . Après une incubation de 30 minutes à 37°C en chambre humide, les bactéries immobilisées sur la lame sont rincées 3 fois avec le tampon NAPB. 50 μl de solution de conjugué DPV~Bodipy ou DPV~TMR ou produit contrôle sont déposés sur les bactéries. Après une incubation de 30 minutes à 37°C en chambre humide et à l'abri de la lumière, les lames sont rincées 3 fois avec le tampon NAPB. Les bactéries peuvent être fixées sur la lame par une incubation de 20 minutes à 37°C à l'abri de la lumière. Une goutte de PBS/Glycérol 50% est déposée sur la lame et recouverte d'une lamelle de verre. Après scellage de la lamelle sur la lame, la fluorescence des bactéries est observée sous microscope optique à épifluorescence Leica (objectif 40X ou 63X à immersion). Les images sont prises avec la caméra numérique Nikon coolpix au zoom maximal et avec l'adaptateur 0.63 X. Une analyse plus détaillée est réalisée au microscope confocal Bio-rad MRC 600 (BIO-RAD Microscience Ltd., Hemel Hempstead, England), équipé d'un microscope optique inversé et d'un objectif à immersion X100. Les bactéries sont visualisées par leur fluorescence après excitation par un laser Krypton/Argon. Plusieurs coupes de bactéries de 0.1 - 0.2μm sont réalisées.

I.8.b) Evaluation de la pénétration des conjugués dans la bactérie à +4°C.

La méthode décrite précédemment (I.8.a) a été modifiée comme suit. Les bactéries immobilisées sur lame de poly-L-Lysine et rincées 3 fois avec le tampon NAPB sont incubées pendant 24 heures à +4°C avant l'addition des conjugués DPV fluorescents pré-incubés à +4°C. Toutes les étapes suivantes sont réalisées à +4°C avec des solutions froides.

I.9) Immuno-marquage de la membrane externe des bactéries : immunofluorescence indirecte.

Les bactéries immobilisées sur lame de poly-L-Lysine sont rincées 3 fois avec du tampon NAPB et incubées 30 minutes à température du laboratoire avec une solution NAPB/SAB 0.05% (Sérum albumine bovine). Les bactéries sont incubées 30 min à la température du laboratoire avec l'anticorps monoclonal de souris anti-endotoxine (Biovalley Cat# C55157; batch# 212529) dilué dans NAPB/SAB 0.05% , lavées plusieurs fois avec NAPB/SAB 0.05% puis incubées 30 minutes à la température du laboratoire, à l'abri de la lumière, avec un deuxième anticorps : anticorps polyclonal de lapin anti-souris conjugué à la tétraméthyl rhodamine (TRITC) (Jackson ImmunoResearch Cat# 315-026-003, batch# 47511) ou à la fluorescéine (FITC) ((Jackson ImmunoResearch Cat# 715-095-150, batch# 51038). Après plusieurs lavages avec le tampon NAPB, une goutte de PBS/Glycérol 50% est déposée sur la lame et recouverte d'une lamelle de verre. Après scellage de la lamelle sur la lame, la fluorescence des bactéries est observée au microscope confocal comme il est décrit précédemment (§ I.8.a).

I.10) Evaluation de l'activité anti-bactérienne des conjugués.

Les concentrations minimales inhibitrices (CMI) sont déterminées par la méthode de microdilution en milieu liquide (NCCLS M7A5) pour l'ensemble des espèces bactériennes en plaque de polystyrène 96 puits.

Une colonie isolée de la bactérie *E. coli* ATCC 25922 ou P. *Aeruginosa* ATCC 27853 est mise en suspension dans 3 à 5ml de milieu de culture Mueller-Hinton (MH) et incubée à 37°C pendant une nuit sous agitation. A partir de cette culture de nuit, une culture en phase exponentielle de croissance de la souche est réalisée; le milieu MH est ensemencé au 1/50è avec la culture de nuit et incubé 2 heures à 37°C sous agitation. La concentration bactérienne est ajustée à $1 \times 10^6$ ufc/ml (Unités Formant Colonies) dans le milieu MH.

50 $\mu$l d'inoculum bactérien sont distribués par puits contenant un volume égal de la solution de conjugué diluée de demi en demi dans le milieu de culture adéquat (0 à 1 $\mu$M). Les cultures sont incubées à 37°C en air ambiant pendant 16 à 20 heures.

La CMI exprimée en $\mu$M est la première concentration ne présentant pas de croissance bactérienne.

II - Résultats.

II.1) Internalisation des conjugués DPV~Bodipy dans la bactérie Gram-négatif.

II.1.a) Evaluation qualitative de l'internalisation des conjugués DPV~Bodipy.

- Evaluation qualitative dans la bactérie *E. coli*.

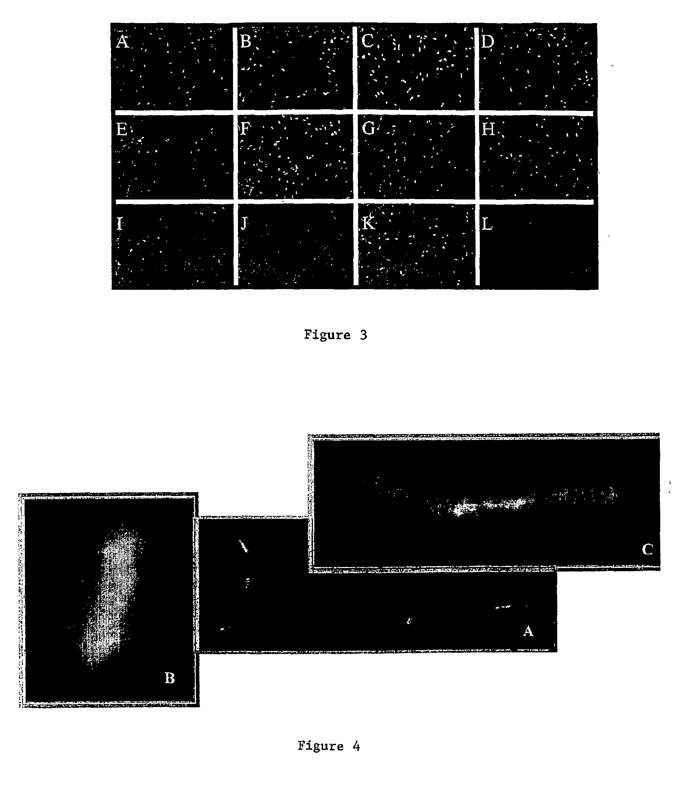

La figure 3 représente l'internalisation des conjugués DPV~Bodipy dans *E. coli*.

Les bactéries immobilisées sur lame de poly-L-Lysine sont incubées avec 1$\mu$M de conjugué DPV~Bodipy pendant 30 minutes à 37°C. Les images de microscopie (microscope optique à épifluorescence, objectif X 63 à immersion) montrent la pénétration du conjugué DPV~Bodipy dans les bactéries vivantes. A: DPV3, B: DPV3.10; C: DPV6; D: DPV7; E: DPV7b; F: DPV15; G: DPV15b; H: DPV1047; I: DPV11; J: DPV12; K: DPV1121; L: DPV19.

Les bactéries *E. coli* sont incubées 30 minutes à 37°C avec 1$\mu$M de conjugué DPV~Bodipy comme il est décrit dans le paragraphe I.8.a. L'internalisation des conjugués DPV~Bodipy fluorescents dans les bactéries non fixées est visualisée sous microscope à épifluorescence. Aucune fluorescence n'est détectée avec le conjugué contrôle Cys-Bodipy. Comme il est montré sur la figure 3, plusieurs conjugués DPV~Bodipy traversent les membranes bactériennes de la bactérie *E. coli* pour s'accumuler dans le cytoplasme bactérien. Les peptides DPV3.10 (Fig. 3B), DPV3 (Fig. 3A), DPV6 (Fig. 3C) et DPV15 (Fig. 3F) sont fortement pénétrant et internalisant de molécules hydrophobes. Avec les peptides DPV11 et DPV12 (Fig. 3I et 3J), nous obtenons des niveaux de fluorescence hétérogènes dans une même population bactérienne. Les propriétés d'internalisation de ces peptides sont plus faibles. Le peptide DPV19 ne pénètre pas dans les bactéries.(Fig. 3L).

Un profil identique d'internalisation des conjugués DPV~Bodipy est observé après fixation des bactéries.

La figure 4 représente l'immuno-marquage de la membrane externe après internalisation du conjugué DPV3~bodipy.

Les bactéries *E. coli* immobilisées sur lame de poly-L-Lysine sont incubées avec 3$\mu$M du conjugué DPV3~Bodipy à 37°C pendant 30 minutes. Après internalisation, la membrane externe des bactéries vivantes est détectée par immuno-marquage en utilisant un anticorps monoclonal de souris anti-endotoxine et un anticorps polyclonal de lapin anti-Ig G de souris couplé au TRITC. La localisation du conjugué DPV3~Bodipy (fluorescence verte) et l'immuno-marquage de la membrane externe (fluorescence rouge) sont observés au microscope confocal. A : Taille originale de l'image ; B et D : Deux agrandissements de bactéries de l'image A.

Afin de confirmer la localisation des conjugués DPV~Bodipy dans le cytoplasme bactérien, les bactéries *E. coli* sont incubées avec 3$\mu$M du conjugué DPV3-Bodipy comme il est décrit dans le paragraphe I.8 et la membrane externe des bactéries vivantes est visualisée par immuno-marquage spécifique comme il est décrit dans le paragraphe I 9. L'endotoxine est un constituant spécifique de la membrane externe des bactéries Gram-négatif. La fluorescence des bactéries est visualisée au microscope confocal (Figure 4). L'internalisation du Bodipy est visualisée par une fluorescence verte et la membrane externe est identifiée par une fluorescence rouge. L'analyse de ces images montre clairement que le peptide DPV3 traverse les membranes externe et interne de la bactérie Gram-négatif *E. coli* et permet l'accumulation du Bodipy dans le cytoplasme bactérien.

- Evaluation qualitative dans la bactérie *P. aeruginosa*.

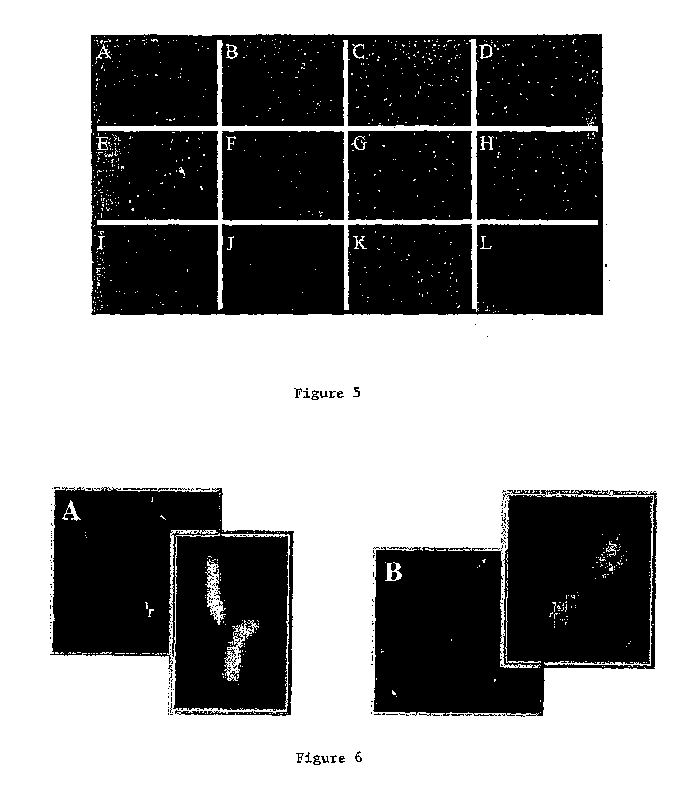

La figure 5 représente l'internalisation des conjugués DPV~Bodipy dans *P.aeruginosa*.

Les bactéries immobilisées sur lame sont incubées avec 1μM de conjugué DPV~Bodipy pendant 30 minutes à 37°C. Les images de microscopie (microscope optique à épifluorescence, objectif X 63 à immersion) montrent la pénétration du conjugué DPV~Bodipy dans les bactéries vivantes. A: DPV3, B: DPV3.10; C: DPV6; D: DPV7; E: DPV7b; F: DPV15; G: DPV15b; H: DPV1047; I: DPV11; J: DPV12; K: DPV1121; L: DPV19.

La figure 6 donne des images de microscopie confocale de *P. aeruginosa*.

Les bactéries sont immobilisées sur lame de poly-L-Lysine et incubées avec 3μM de conjugué DPV3~Bodipy (A) ou de conjugué DPV7~Bodipy (B) à 37°C pendant 30 min puis fixées sur la lame. Les bactéries sont observées au microscope confocal. Un agrandissement de l'image originale des bactéries est présenté.

Une même évaluation qualitative est réalisée sur *P aeruginosa*. La figure 5 montre l'internalisation des conjugués dans les bactéries après 30 minutes d'incubation. Les propriétés d'internalisation des DPV sont identiques à celles observées pour *E. coli* excepté pour les peptides DPV7b et DPV6 qui semblent être plus internalisants chez *P. aeruginosa*. L'observation au microscope confocal (Figure 6) des bactéries incubées avec les peptides DPV3 ou DPV7b montre que ces peptides ont la capacité de traverser la membrane externe et permettre l'accumulation du Bodipy dans le cytoplasme bactérien.

II.1.b) Classification des DPVs.

Comme il est montré sur les figures 3 et 5, le niveau d'accumulation des conjugués DPV~Bodipy dans le cytoplasme bactérien est variable selon le DPV et selon la souche de bactérie. De façon générale, l'internalisation des DPV est quasiment identique pour les deux souches de bactéries étudiées. Les peptides DPV peuvent être classés en trois groupes majeures :

- DPV3, DPV3.10 : internalisation élevée
- DPV6, DPV7, DPV7b, DPV15 : internalisation moyenne
- DPV15b, DPV1047, DPV1121 : internalisation faible

Les peptides DPV3.10, DPV3, DPV6, DPV7 et DPV7b ont précédemment été décrits comme des peptides à localisation cytoplasmique dans les cellules eucaryotes (demande de brevet internationale PCT publiée sous le No. WO 01/64738) quand ils sont couplés chimiquement à la protéine peroxydase ou à des IgG. A l'opposé, les peptides DPV15, DPV15b, DPV1047 et DPV1121 ont été décrits comme des peptides à localisation nucléaire. Il est important de noter que le niveau d'internalisation des DPV « nucléaires » est plus faible que celui des DPV « cytoplasmiques ». Les peptides ayant un tropisme cytoplasmique sont les plus internalisants dans la bactérie.

Comme l'indique le tableau 1 ci-après, les peptides DPV19, DPV11 et DPV12 n'ont pas de propriété d'internalisation dans la cellule eucaryote. La même propriété est observée avec les cellules procaryotes, comme les bactéries Gram-négatif.

Tableau 1 : Evaluation qualitative de l'interna-lisation des conjugués DPV~Bodipy dans la bactérie Gram-négatif.

Tableau 1

|  | *E. coli* | *P. aeruginosa* |
|---|---|---|
| DPV3~Bodipy | +++ | +++ |
| DPV3.10~Bodipy | ++++ | ++++ |
| DPV6~Bodipy | ++ | +++ |
| DPV7~Bodipy | ++ | ++ |
| DPV7b~Bodipy | + | +++ |

(suite)

|  | *E. coli* | *P. aeruginosa* |
| --- | --- | --- |
| DPV15~Bodipy | ++ | ++ |
| DPV15b~Bodipy | + | + |
| DPV1047~Bodipy | + | + |
| DPV1121~Bodipy | + | + |
| DPV19~Bodipy | - | - |
| DPV11~Bodipy | +/- | +/- |
| DPV12~Bodipy | - | - |

II.1.c) <u>Etude de l'effet de la poly-L-Lysine (support des bactéries) sur l'internalisation</u>.

Afin de confirmer les résultats précédents et d'évaluer une éventuelle interférence de la poly-L-Lysine avec l'internalisation des conjugués, les bactéries *E. coli* sont incubées avec les conjugués DPV~Bodipy pendant 30 minutes à 37°C puis lavées de façon extensive avec le tampon NAPB avant d'être immobilisées et fixées ou non sur lame de poly-L-Lysine. La localisation des conjugués est visualisée au microscope optique à épifluorescence ou confocal. Les propriétés d'internalisation des différents DPV ne diffèrent pas des résultats obtenus précédemment. La Poly-L-Lysine n'a pas d'effet sur la capacité du DPV à traverser les membranes bactériennes et à pénétrer dans la bactérie.

II.1.d) <u>Influence de la température sur le niveau d'internalisation</u>.

Afin d'expliquer le mécanisme d'internalisation précédemment observé, la capacité des DPV à internaliser à +4°C a été analysée. Les bactéries *E. coli* en phase exponentielle de croissance sont immobilisées sur lame de poly-L-Lysine puis incubées pendant 24 heures à +4°C afin d'abolir le métabolisme énergétique de la bactérie. Les bactéries sont ensuite incubées avec $3\mu$M de DPV7~Bodipy ou Cyst~Bodipy (contrôle) pendant 30 minutes à +4°C comme il est décrit dans le paragraphe I.8.b) et lavées de manière extensive avant d'être visualisées au microscope optique à épifluorescence et confocal. Pour comparer les niveaux d'internalisation à 37°C et à +4°C, la même expérience est conduite à 37°C comme il est décrit dans le paragraphe I.8.a.
Le niveau d'internalisation du DPV7 dans la bactérie est identique quelque soit la température de l'expérience. Il semble donc que l'internalisation du conjugué DPV7~Bodipy dans *E. coli* n'est pas un mécanisme énergie-dépendant. Le phénomène est probablement une translocation passive au travers des membranes bactériennes.

II.2) <u>Internalisation du conjugué DPV3~TMR dans *E. coli.*</u>

Nous avons montré que certains peptides DPV peuvent traverser la membrane externe des bactéries Gram-négatif et pénétrer dans la bactérie pour internaliser un composé hydrophobe comme le Bodipy qui est normalement exclu par cette membrane externe.
Afin de valider les résultats obtenus précédemment et exclure une quelconque influence du traceur fluorescent Bodipy sur l'internalisation, des expériences identiques ont été menées avec un deuxième traceur fluorescent hydrophobe, le TMR. Le TMR diffère du Bodipy par des propriétés physico-chimiques comme sa structure ou la présence d'une charge positive (Fig. 2).

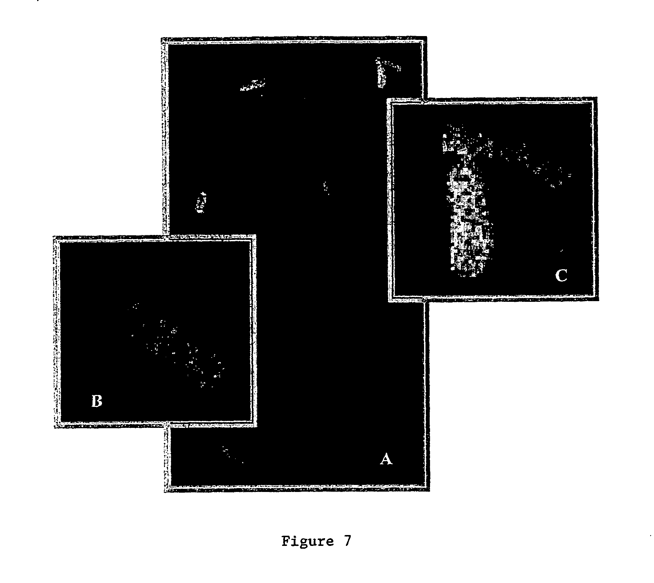

La figure 7 représente l'internalisation du conjugué DPV3~TMR dans *E. coli.*
Les bactéries *E. coli* sont immobilisées sur lame de poly-L-Lysine et incubées avec $1\mu$M du conjugué DPV3-TMR à 37°C pendant 30 min. Après internalisation, la membrane externe des bactéries vivantes est détectée par immuno-marquage en utilisant un anticorps monoclonal de souris anti-endotoxine et un anticorps polyclonal de lapin anti-Ig G de souris couplé au FITC. La localisation du conjugué DPV3-TMR (fluorescence rouge) et l'immuno-marquage de la membrane externe (fluorescence verte) sont observés au microscope confocal. A : Taille originale de l'image ; B et D : Deux agrandissements de bactéries de l'image A.
L'internalisation du conjugué DPV3 TMR est évaluée sur des bactéries *E. coli* immobilisées sur lame de poly-L-Lysine ou en suspension. Les bactéries sont incubées avec $1\mu$M de conjugué DPV3-TMR ou le contrôle Cyst~TMR pendant 30 minutes à 37°C, puis fixées ou non sur lame avant d'être visualisées au microscope optique à épifluorescence. Quelque soit le protocole d'internalisation utilisé, aucune fluorescence n'est détectée avec le conjugué contrôle tandis que le DPV3-TMR est visualisé par la fluorescence rouge de la bactérie.
Afin de confirmer l'internalisation du conjugué DPV3~TMR, les bactéries sont immobilisées sur lame de poly-L-Lysine et incubées avec 1μM de conjugué à 37°C pendant 30 minutes. L'immuno-marquage de la membrane externe est réalisé en utilisant l'anticorps monoclonal de souris anti-endotoxine et un anticorps polyclonal de lapin anti-IgG de souris couplé au FITC. La localisation du conjugué DPV3~TMR est observée au microscope confocal (Figure 7). Le conjugué DPV3~TMR traverse la membrane externe, pénètre dans la bactérie et s'accumule dans le cytoplasme. Ce résultat est identique à ceux obtenus avec le traceur fluorescent Bodipy.

II.3) Activité anti-bactérienne des conjugués DPV.

Afin de déterminer que l'internalisation des conjugués DPV n'induit pas la mort des bactéries, les 12 conjugués DPV~Bodipy et le conjugué DPV3~TMR sont testés pour leur activité anti-bactérienne comme il est décrit dans le paragraphe I.10) Aucun des conjugués testés n'a montré une activité anti-bactérienne sur *E. coli* aux concentrations utilisés dans les expériences d'internalisation. Cette expérience montre que l'internalisation des conjugués n'affecte pas la viabilité bactérienne. Le mécanisme d'internalisation dans la bactérie n'est pas toxique.

III - Internalisation d'un conjugué DPV~Antibiotique (exemple : DPV~Erythromycine)

III.1) Synthèse d'un conjugué DPV~Antibiotique

III.1.a) Activation de l'Erythromycine par un cross linker hétérobifonctionnel

Une solution de maleimidocaproic acid (MIC) (2,8 équivalents) et de dicyclohexylcarbodiimide (DCC) (2,8 équivalents) dans de la diméthylformamide (DMF) est agitée la nuit à 0°C sous argon et à l'abri de la lumière. Le précipité formé (dicyclohexylurée) est éliminé par filtration, lavé avec de la DMF puis filtré à nouveau. Une solution d'antibiotique (1,0 équivalent) et de pyridine (5,0 équivalents) dans de la DMF est agitée jusqu'à dissolution complète. On additionne sur cette solution le filtrat obtenu ci-dessus ; le mélange est agité 1 heure à température ambiante.
La solution est reprise dans de l'eau distillée, lavée 4 fois avec du dichloromethane (DCM). Les phases organiques obtenues sont rassemblées et lavées successivement avec de l'acide chlorhydrique (HCl) 0,1 N, deux fois avec du carbonate de disodium ($Na_2CO_3$), trois fois avec de l'eau ($H_2O$). Après séchage sur du sulfate de magnésium ($MgSO_4$) et concentration, le brut réactionnel est purifié par chromatographie flash sur silice (éluant $CH_2Cl_2$ / MeOH).

III.1.b) Couplage de l'Erythromycine activée avec un peptide pénétrant DPV

Un peptide pénétrant (1,0 équivalent) dans une solution tampon de phosphate de sodium ($NaH_2PO_4$/$Na_2HPO_4$) à 10 mM et pH 7,1 est agité cinq minutes à température ambiante sous argon et à l'abri de la lumière. Puis, l'antibiotique activé (1,5 à 2,0 équivalents), dissous dans le minimum de DMF, est ajouté. La solution est agitée jusqu'à transformation complète du peptide (suivi HPLC). De l'eau distillée est ensuite ajoutée et la phase aqueuse est extraite trois fois avec le même volume de dichlorométhane afin d'éliminer l'excès d'antibiotique activé. La phase aqueuse est ensuite lyophilisée et le solide obtenu est purifié par HPLC préparative. Le conjugué antibiotique-peptide pénétrant est ainsi isolé avec des rendements compris entre 45 et 100% et des puretés supérieures à 90%.

III.2) Evaluation de l'activité antimicrobienne des conjuguées DPV~Erythromycine.

Les concentrations minimales inhibitrices (CMI) de conjugués sont déterminées par la méthode de microdilution en milieu liquide selon les normes NCCLS-M7A5 (National Committee for Clinical Laboratory Standards - Document M7A5) pour l'ensemble des espèces bactériennes en plaque de polystyrène 96 puits.

Protocol :

Une colonie isolée d'une bactérie (par exemple *E. coli* ou *P. Aeruginosa*) est mise en suspension dans 3 à 5ml de milieu de culture Mueller-Hinton (MH) et incubée à 37°C pendant une nuit sous agitation. A partir de cette culture de nuit, une culture en phase exponentielle de croissance de la souche est réalisée; le milieu MH est ensemencé au 1/50è avec la culture de nuit et incubé 2 heures à 37°C sous agitation. La concentration bactérienne est ajustée à $1\times10^6$ ufc/ml (Unités Formant Colonies) dans le milieu MH.
50 μl d'inoculum bactérien sont distribués par puits contenant un volume égal de la solution de conjugué diluée de demi en demi dans le milieu de culture adéquat (512 à 0,5 μg/ml). Les cultures sont incubées à 37°C en air ambiant pendant 16 à 20 heures.
La CMI exprimée en μg/ml (Unités Internationales) est la première concentration ne présentant pas de croissance bactérienne. La détermination de la concentration minimale bactéricide (CMB) est réalisée après lecture des plaques de CMI. La CMB est la plus faible concentration de conjugué qui inhibe toute croissance bactérienne sur la gélose de repiquage (<0,1% de survivants).

IV - Evaluation de l'activité antibactérienne de l'association d'un peptide DPV et de l'érythromycine A par la méthode dite de l'échiquier

IV-1) Matériels et Méthodes

    Peptides choisis : DPV3 et DPV3.10
    Composé antibactérien : Erythromycine (Sigma E0774)
    Souches bactériennes : E. coli ATCC 25922 et P. aeruginosa ATCC 27853.

Cette méthode est réalisée en microplaques de polystyrène 96 puits dans le milieu de culture Mueller-Hinton (MH) et consiste à exposer une suspension bactérienne à différentes concentrations de peptide DPV et d'érythromycine, employés seuls ou en association.

Les concentrations finales choisies d'érythromycine et de peptide s'échelonnent respectivement de 256 à 4µg/ml et de 256 à 2µg/ml. Les gammes de dilutions sont préparées selon une progression géométrique de raison 2

25µl des solutions de produits en milieu MH dont la concentration est quatre fois plus élevée que la concentration finale désirée ou 25µl de milieu MH (pour les rangées 0) sont distribués dans les puits selon le schéma ci-dessous (tableau 2), afin d'obtenir un volume final de 50µl par puits:

Tableau 2 : distribution des solutions de produit en milieu MH (les puits marqués X n'ont pas été utilisés)

### Tableau 2

| | Concentration finale de peptide DPV (µg/ml) | | | | | | | | | | |
|---|---|---|---|---|---|---|---|---|---|---|---|
| | 0 | 256 | 128 | 64 | 32 | 16 | 8 | 4 | 2 | | |
| Conc 0 | | | | | | | | | | X | X |
| 256 | | | | | | | | | | X | X |
| 128 | | | | | | | | | | X | X |
| 64 | | | | | | | | | | X | X |
| 32 | | | | | | | | | | X | X |
| 16 | | | | | | | | | | X | X |
| 8 | | | | | | | | | | X | X |
| 4 | | | | | | | | | | X | MH |

Une colonie isolée de la bactérie *E. coli* ATCC 25922 ou *P. aeruginosa* ATCC 27853 est mise en suspension dans 3 à 5ml de milieu de culture MH et incubée à 37°C pendant une nuit sous agitation. A partir de cette culture de nuit, une culture en phase exponentielle de croissance de la souche est réalisée ; le milieu MH est ensemencé au 1/50è avec la culture de nuit et incubé 2 heures à 37°C sous agitation. La concentration bactérienne est ajustée à $5 \times 10^5$ - $10^6$ ufc/ml (Unités Formant Colonies) dans le milieu MH. 50 µl d'inoculum bactérien sont distribués par puits contenant un volume égal de la solution de peptide et/ou d'érythromycine. La CMI du peptide et de l'éryhromicine est déterminée comme la concentration la plus faible qui provoque l'absence de croissance des bactéries (absence de turbidité) après 18 heures de culture dans une étuve à 37°C. La CMI est exprimée en µg/ml (mg/l). Pour chaque rangée de puits, les premiers puits contenant l'association du peptide DPV et de l'érythromycine et ne présentant aucune croissance visible sont notés afin de calculer pour chacun l'indice de fraction de la concentration inhibitrice (FIC) en utilisant la formule

suivante :

$$FIC = (CMI \text{ du peptide avec l'érythromycine}/CMI \text{ du peptide seul}) + (CMI \text{ de l'érythromycine avec le peptide}/ CMI \text{ de l'érythromycine seule}).$$

Cet indice permet de quantifier l'association. Un indice inférieur ou égal à 0,5 indique une synergie, un indice supérieur à 2 un antagonisme, Un effet d'addition est indiqué par une FIC comprise entre 0,5 et 1, et un effet d'indifférence, par une FIC dont les valeurs sont comprises entre 1 et 2.

IV.2) Résultats

Les bactéries Gram-négatif telles que *E. coli* et *P. aeruginosa* sont résistantes aux antibiotiques de la famille des macrolides tel que l'érythromycine du fait de la non-pénétration de ces antibiotiques au travers de la membrane externe de la bactérie. Afin d'évaluer les propriétés internalisantes des peptides DPVs mises en évidence précédemment et leur capacité à faciliter la pénétration d'un antibiotique de la famille des macrolides, l'activité antibactérienne de l'érythromycine sur les bactéries *E. coli* et *P. aeruginosa* a été évaluée en association avec le peptide DPV3 ou DPV3.10 selon la méthode dite de l'échiquier.

L'effet synergique de l'association des peptides DPV3 et 3.10 avec l'érythromycine est montré tableaux 3 et 4. En présence du DPV3, un effet synergique avec l'érythromycine est observé uniquement sur *E. coli* ce qui montre que ce peptide permet l'entrée de l'érythromycine dans *E coli.* L'association DPV3.10 avec l'érythromycine est synergique sur les deux souches bactériennes. Le peptide DPV3.10 à la concentration non toxique de $32\mu g/ml$ permet la pénétration (l'internalisation) de l'érythromycine.

Tableau 3 : Détermination des CMIs et FICs de l'érythromycine en association avec le DPV3

Tableau 3

| Bactéries | CMI ($\mu$g/ml) | | | Index FIC | Synergie |
|---|---|---|---|---|---|
| | Erythromycine seule | DPV3 seul | Erythromycine associée à DPV3* | | |
| *E. coli* | 256 | 256 | 64 (64) | 0.5 | Oui |
| *P. aeruginosa* | 256 | 256 | 64 (64) | 0.75 | Non |
| | | | 4 (128) | 0.52 | Non |
| * les valeurs entre parenthèses sont les concentrations ($\mu$g/ml) de DPV3 ajouté | | | | | |

Tableau 4 : Détermination des CMIs et FICs de l'érythromycine en présence de peptide DPV3.10

Tableau 4

| Bactéries | CMI ($\mu$g/ml) | | | Index FIC | Synergie |
|---|---|---|---|---|---|
| | Erythromycine seule | DPV3 seul | Erythromycine associée à DPV3* | | |
| *E. coli* | 256 | 128 | 64 (32) | 0.5 | Oui |
| *P. aeruginosa* | 256 | 256 | 64 (32) | 0.375 | Oui |
| * les valeurs entre parenthèses sont les concentrations ($\mu$g/ml) de DPV3.10 ajouté | | | | | |

## Revendications

1. Utilisation d'au moins un peptide de 10 à 25 résidus d'acide aminé lié par covalence à un composé anti-bactérien, le(s)dit(s) peptide comprenant :

   i) deux domaines chargés positivement à pH neutre constitué de 3 à 9 résidus d'acide aminé dont les deux tiers au moins sont des acides aminés cationiques,
   ii) entre lesdits domaines chargés positivement, un groupe de deux à trois résidus d'acide aminé non cationique,
   iii) à l'une et/ou l'autre des extrémités N ou C terminale du peptide, un groupe de 0 à 10 résidus d'acide aminé choisis dans le groupe comprenant des acides aminés non hydrophobes et des acides aminés chargés positivement, mais dans le cas d'un résidu d'acide aminé chargé positivement celui-ci n'est pas directement adjacent aux domaines chargés positivement,

   pour la préparation d'une composition pharmaceutique destinée au traitement d'une infection par des bactéries gram-négatif

2. Utilisation selon la revendication 1, dans laquelle ledit peptide est lié à l'agent antibactérien par l'intermédiaire d'un bras espaceur.

**3.** Utilisation selon la revendication 1 ou 2, dans laquelle ledit peptide comprend à l'une et/ou l'autre des extrémités N ou C terminale du peptide, un groupe de 0 à 5 résidus d'acide aminé choisis dans le groupe comprenant des acides aminés non hydrophobes et des acides aminés chargés positivement, mais dans le cas d'un résidu d'acide aminé chargé positivement celui-ci n'est pas directement adjacent aux domaines chargés positivement

**4.** Utilisation selon l'une quelconque des revendications 1 à 3, d'au moins un peptide dans lequel

 (i) les acides aminés cationiques des deux domaines chargés positivement sont choisis dans le groupe comprenant l'arginine et la lysine, et dans lequel
 (ii) les acides aminés non cationiques du groupe entre lesdits domaines chargés positivement sont des acides aminés non hydrophobes, choisis par exemple dans le groupe comprenant l'acide glutamique, la serine, la glycine, la leucine, la glutamine.

**5.** Utilisation selon l'une des revendications 1 à 4, dans laquelle le peptide est choisi dans le groupe comprenant les séquences suivantes : SEQ ID NO.1, SEQ ID NO.2, SEQ ID NO.3, SEQ ID NO.4, SEQ ID NO.5, SEQ ID NO.6, SEQ ID NO.7, SEQ ID NO.8, SEQ ID NO.9, SEQ ID NO.11.

**6.** Utilisation selon l'une quelconque des revendications 1 à 5, dans laquelle le peptide est choisi dans le groupe comprenant les séquences suivantes : SEQ ID NO.1, SEQ ID NO.2, SEQ ID NO.3, SEQ ID NO.4, SEQ ID NO.5, SEQ ID NO.6, SEQ ID NO.7.

**7.** Utilisation selon l'une quelconque des revendications 1 à 6, dans laquelle le composé anti-bactérien est choisi parmi ceux présentant des propriétés physico-chimiques le rendant incapable de traverser la membrane des bactéries gram négatif.

**8.** Utilisation selon l'une quelconque des revendications précédentes, dans laquelle le composé anti-bactérien est hydrophobe.

**9.** Utilisation selon l'une quelconque des revendications précédentes, dans laquelle le composé anti-bactérien est choisi dans le groupe comprenant les composés suivants : les antibiotiques de la famille de macrolides, des ketolides comme l'erythromycine, la clarithromycine, l'azithromycine, la télithromycine.

**10.** Utilisation selon l'une quelconque des revendications 1 à 9, dans laquelle la composition pharmaceutique antibactérienne comprend un produit de formule (I) suivante :

$$(A\text{-})_m(X)_p(\text{-}P)_n \qquad (I)$$

dans laquelle : A est le reste d'un composé anti-bactérien, P est le reste d'une peptide, tels que définis dans les revendications précédentes, et X représente soit une liaison covalente entre A et P, soit un bras espaceur reliant au moins un reste A à au moins un reste P, m est un nombre entier pouvant aller de 1 à 3, n est un nombre entier pouvant aller de 1 à 3, et p représente zéro ou un nombre entier au plus égal au plus grand des nombres m et n.

**11.** Composition antibactérienne **caractérisée en ce qu'**elle comprend au moins un peptide lié par covalence un composé anti-bactérien, éventuellement par l'intermédiaire d'un bras espaceur, et **en ce que** le(s)dit(s) peptide est choisi dans le groupe comprenant les séquences suivantes : SEQ ID NO.1, SEQ ID NO.2, SEQ ID NO.3, SEQ ID NO.4, SEQ ID NO.5, SEQ ID NO.6, SEQ ID NO.7, SEQ ID NO.8, SEQ ID NO.9, SEQ ID NO.11.

**12.** Composition antibactérienne selon la revendication 11, dans laquelle la composition comprend l'association d'au moins un peptide et d'un composé anti-bactérien liés par covalence éventuellement par l'intermédiaire d'un bras espaceur, ledit peptide étant choisi dans le groupe comprenant les séquences suivantes : SEQ ID NO.1, SEQ ID NO.2, SEQ ID NO.3, SEQ ID NO.4, SEQ ID NO.5, SEQ ID NO.6, SEQ ID NO.7.

**13.** Un produit de formule (I) suivante :

$$(A\text{-})_m(X)_p(\text{-}P)_n \qquad (I)$$

dans laquelle A, X, m, p et n sont définis comme dans la revendication 10, et P est le reste d'une peptide choisi dans le groupe comprenant les séquences suivantes : SEQ ID NO.1, SEQ ID NO.2, SEQ ID NO.3, SEQ ID NO.4, SEQ ID NO.5, SEQ ID NO.6, SEQ ID NO.7, SEQ ID NO.8, SEQ ID NO.9, SEQ ID NO.11.

**14.** Un produit de formule (I) suivante selon la revendication 13:

$$(A-)_m(X)_p(-P)_n \qquad (I)$$

dans laquelle A, X, m, p et n sont définis comme dans la revendication 10, et P est le reste d'une peptide choisi dans le groupe comprenant les séquences suivantes : SEQ ID NO.1, SEQ ID NO.2, SEQ ID NO.3, SEQ ID NO.4, SEQ ID NO.5, SEQ ID NO.6, SEQ ID NO.7.

**Claims**

**1.** The utilisation of at least one peptide of 10 to 25 amino acid residues linked by covalence to an antibacterial component, said peptide(s) including:

i) two positively charged domains at a neutral pH of 3 to 9 amino acid residues, two thirds of which at least are cationic amino acids,
ii) between said positively charged domains, a group of two to three non cationic amino acid residues,
iii) at one and/or the other of the N or C terminations of the peptide, a group of 0 to 10 amino acid residues selected in the group including non hydrophobic amino acids and positively charged amino acids, but in the case of a positively charged amino acid residue, the latter is not directly adjoining the positively charged domains,

for the preparation of a pharmaceutical composition intended for the treatment of an infection caused by gram-negative bacteria.

**2.** The utilisation according to claim 1, wherein said peptide is linked to the antibacterial agent through a spacer arm.

**3.** The utilisation according to claim 1 or 2, wherein said peptide includes at one and/or the other of the N or C terminations of the peptide, a group of 0 to 5 amino acid residues selected in the group including non hydrophobic amino acids and positively charged amino acids, but in the case of a positively charged amino acid residue, the latter is not directly adjoining the positively charged domains.

**4.** The utilisation according to any one of claims 1 to 3, of at least one peptide wherein

(i) the cationic amino acids of both positively charged domains are selected in the group including arginine and lysine and wherein
(ii) the non cationic amino acids of the group between said positively charged domains are non hydrophobic amino acids, selected for example in the group containing glutamic acid, serine, glycine, leucine, glutamine.

**5.** The utilisation according to one of claims 1 to 4, wherein the peptide is selected in the group including the following sequences: SEQ ID NO.1, SEQ ID NO.2, SEQ ID NO.3, SEQ ID NO.4, SEQ ID NO.5, SEQ ID NO.6, SEQ ID NO. 7, SEQ ID NO.8, SEQ ID NO.9, SEQ ID NO.11.

**6.** The utilisation according to any one of claims 1 to 5, wherein the peptide is selected in the group including the following sequences: SEQ ID NO.1, SEQ ID NO.2, SEQ ID NO.3, SEQ ID NO.4, SEQ ID NO.5, SEQ ID NO.6, SEQ ID NO.7.

**7.** The utilisation according to any one of claims 1 to 6, wherein the antibacterial compound is selected among those having physical-chemical properties making it incapable of going through the membrane of the gram negative bacteria.

**8.** The utilisation according to any one of the preceding claims, wherein the antibacterial compound is hydrophobic.

**9.** The utilisation according to any one of the preceding claims, wherein the antibacterial compound is selected in the group including the following compounds: antibiotics of the family of macrolides, ketolides such as erythromycin, clarithromycin, azithromycin, telithromycin.

**10.** The utilisation according to any one of claims 1 to 9, wherein the antibacterial pharmaceutical composition includes a product having the following formula (I):

$$(A-)_m(X)_p(-P)_n \qquad (I)$$

wherein: A is the remaining part of an antibacterial compound, P is the remaining part of a peptide such as defined in the preceding claims, and X is either a covalent link between A and P, or a spacer arm linking at least a remaining part A to at least a remaining part P, m is an integer from 1 to 3, n is an integer from 1 to 3, and p is zero or an integer at most equal to the greater number among m and n.

**11.** An antibacterial composition, **characterised in that** it includes at least one peptide linked by covalence to an antibacterial compound, possibly through a spacer arm, and **in that** said peptide or peptides is or are selected in the group including the following sequences: SEQ ID NO.1, SEQ ID NO.2, SEQ ID NO.3, SEQ ID NO.4, SEQ ID NO.5, SEQ ID NO.6, SEQ ID NO.7, SEQ ID NO.8, SEQ ID NO.9, SEQ ID NO.11.

**12.** An antibacterial composition according to claim 11, wherein the composition includes the association of at least one peptide and one antibacterial compound linked by covalence possibly through a spacer arm, said peptide being selected in the group including the following sequences: SEQ ID NO.1, SEQ ID NO.2, SEQ ID NO.3, SEQ ID NO. 4, SEQ ID NO.5, SEQ ID NO.6, SEQ ID NO.7.

**13.** A product having the following formula (I):

$$(A-)_m(X)_p(-P)_n \qquad (I)$$

wherein A, X, m, p and n are defined as in claim 10, and P is the remaining part of a peptide selected in the group including the following sequences: SEQ ID NO.1, SEQ ID NO.2, SEQ ID NO.3, SEQ ID NO.4, SEQ ID NO.5, SEQ ID NO.6, SEQ ID NO.7, SEQ ID NO.8, SEQ ID NO.9, SEQ ID NO.11.

**14.** A product having the following formula (I) according to claim 13:

$$(A-)_m(X)_p(-P)_n \qquad (I)$$

wherein A, X, m, p and n are defined as in claim 10, and P is the remaining part of a peptide selected in the group comprising the following sequences: SEQ ID NO.1, SEQ ID NO.2, SEQ ID NO.3, SEQ ID NO.4, SEQ ID NO.5, SEQ ID NO.6, SEQ ID NO.7.

**Patentansprüche**

**1.** Verwendung von wenigstens einem per Kovalenz an eine antibakterielle Komponente gebundenen Peptid mit 10 bis 25 Aminosäureresten, wobei das / die genannte(n) Peptid(e) Folgendes umfasst / umfassen:

i) zwei positiv geladene Bereiche mit neutralem ph-Wert, bestehend aus 3 bis 9 Aminosäureresten, von denen wenigstens zwei Drittel kationische Aminosäuren sind,
ii) zwischen den genannten positiv geladenen Bereichen eine Gruppe von zwei bis drei nicht kationischen Aminosäureresten,
iii) an dem einen und / oder dem anderen Ende N oder Terminal C des Peptids eine Gruppe von 0 bis 10 Aminosäureresten, die aus der Gruppe ausgewählt wurden, die nicht hydrophobe Aminosäure und positiv geladene Aminosäuren umfassen, aber in dem Fall eines positiv geladenen Aminosäurerestes, ist dieser nicht direkt an den positiv geladenen Bereichen anliegend,

für die Zubereitung einer pharmazeutischen Zusammensetzung, die zur Behandlung einer Infektion durch gram-negative Bakterien bestimmt ist.

**2.** Verwendung gemäß Anspruch 1, in der das genannte Peptid mit dem antibakteriellen Wirkstoff über einen Abstandshalterarm verbunden ist.

**3.** Verwendung gemäß Anspruch 1 oder 2, in dem das genannte Peptid das eine und / oder das andere der Enden N oder Terminal C des Peptids umfasst, eine Gruppe von 0 bis 5 Aminosäureresten, die aus der Gruppe ausgewählt sind, die nicht hydrophobe Aminosäuren und positiv geladene Aminosäuren umfasst, aber in dem Fall eines positiv geladenen Aminosäurerestes ist dieser nicht direkt an den positiv geladenen Bereichen anliegend.

**4.** Verwendung wenigstens eines Peptides gemäß Anspruch 1 bis 3, in dem

(i) die kationischen Aminosäuren der zwei positiv geladenen Bereiche aus der Gruppe ausgewählt sind, die Arginin und Lysin umfassen, und in der

(ii) die nicht kationischen Aminosäuren der Gruppe zwischen den genannten positiv geladenen Bereichen nicht hydrophobe Aminosäuren sind, die zum Beispiel aus der Gruppe ausgewählt sind, die Glutaminsäure, Serin, Glycerin, Leucin, Glutamin umfasst.

**5.** Verwendung gemäß Anspruch 1 bis 4, in der das Peptid aus der Gruppe ausgewählt ist, die die folgenden Sequenzen umfasst: SEQ ID NR. 1, SEQ ID NR. 2, SEQ ID NR. 3, SEQ ID NR. 4, SEQ ID NR. 5, SEQ ID NR. 6, SEQ ID NR. 7, SEQ ID NR. 8, SEQ ID NR. 9, SEQ ID NR. 11.

**6.** Verwendung gemäß Anspruch 1 bis 5, in der das Peptid aus der Gruppe ausgewählt ist, die die folgenden Sequenzen umfasst: SEQ ID NR. 1, SEQ ID NR. 2, SEQ ID NR. 3, SEQ ID NR. 4, SEQ ID NR. 5, SEQ ID NR. 6, SEQ ID NR. 7.

**7.** Verwendung gemäß Anspruch 1 bis 6, in der die antibakterielle Komponente aus denen ausgewählt ist, die physikalisch-chemische Eigenschaften aufweisen, die sie unfähig machen, die Membran der gram-negativen Bakterien zu durchqueren.

**8.** Verwendung gemäß einem der vorherigen Ansprüche, in der die antibakterielle Komponente hydrophob ist.

**9.** Verwendung gemäß einem der vorherigen Ansprüche, in der die antibakterielle Komponente aus der Gruppe ausgewählt ist, die die folgenden Komponenten umfasst: die Antibiotika der Familie der Makroliden, der Ketoliden, wie Erythromycin, Clarithromycin, Azithromycin, Telithromycin.

**10.** Verwendung gemäß Anspruch 1 bis 9, in der die pharmazeutische antibakterielle Komponente ein Produkt der folgenden Formel (I) umfasst:

$$(A-)_m(X)_p(-P)_n \qquad (I)$$

in der: A der Rest einer antibakteriellen Komponente ist, P der Rest eines Peptids ist, wie sie in einem der vorherigen Ansprüche definiert werden, und X entweder eine kovalente Bindung zwischen A und P darstellt oder einen Abstandshalterarm, der wenigstens einen Rest A mit wenigstens einem Rest P verbindet, m eine ganze Zahl ist, die von 1 bis 3 reichen kann, n eine ganze Zahl ist, die von 1 bis 3 reichen kann, und p Null oder eine ganze Zahl darstellt, die maximal gleich der größeren der Zahlen m und n ist.

**11.** Antibakterielle Verbindung, **dadurch gekennzeichnet, dass** sie wenigstens ein Peptid umfasst, das per Kovalenz, eventuell mit einem Abstandshalterarm mit einer antibakteriellen Komponente verbunden ist, und dass das / die genannte(n) Peptid(d) aus der Gruppe ausgewählt ist / sind, die die folgenden Segmente umfasst: SEQ ID NR. 1, SEQ ID NR. 2, SEQ ID NR. 3, SEQ ID NR. 4, SEQ ID NR. 5, SEQ ID NR. 6, SEQ ID NR. 7, SEQ ID NR. 8, SEQ ID NR. 9, SEQ ID NR. 11.

**12.** Antibakterielle Verbindung gemäß Anspruch 11, in der die Verbindung die Assoziation von wenigstens einem Peptid und einer antibakteriellen Komponente umfasst, die per Kovalenz, eventuell über einen Abstandshalterarm, verbunden sind, wobei das genannte Peptid aus der Gruppe ausgewählt ist, die die folgenden Sequenzen umfasst: SEQ ID NR. 1, SEQ ID NR. 2, SEQ ID NR. 3, SEQ ID NR. 4, SEQ ID NR. 5, SEQ ID NR. 6, SEQ ID NR. 7.

**13.** Ein Produkt mit der folgenden Formel (I):

$$(A-)_m(X)_p(-P)_n \qquad (I)$$

in der A, X, m, p, und n wie in Anspruch 10 definiert sind und P der Rest eines Peptids ist, das aus der Gruppe ausgewählt ist, die die folgenden Sequenzen umfasst: SEQ ID NR. 1, SEQ ID NR. 2, SEQ ID NR. 3, SEQ ID NR.

4, SEQ ID NR. 5, SEQ ID NR. 6, SEQ ID NR. 7, SEQ ID NR. 8, SEQ ID NR. 9, SEQ ID NR. 11.

**14.** Ein Produkt mit der folgenden Formel (I) gemäß Anspruch 13:

$$(A-)_m(X)_p(-P)_n \qquad (I)$$

in der A, X, m, p, und n wie in Anspruch 10 definiert sind und P der Rest eines Peptids ist, das aus der Gruppe ausgewählt ist, die die folgenden Sequenzen umfasst: SEQ ID NR. 1, SEQ ID NR. 2, SEQ ID NR. 3, SEQ ID NR. 4, SEQ ID NR. 5, SEQ ID NR. 6, SEQ ID NR. 7.

Figure 1

Figure 2

Figure 3

Figure 4

Figure 5

Figure 6

Figure 7

**EP 1 653 989 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0164738 A
- WO 0164738 PCT
- FR 0309962
- EP 03292030 A

**Littérature non-brevet citée dans la description**

- **Park CB ; Kim HS ; Kim SC.** *Biochem Biophys Res Commun.,* 06 Mars 1998, vol. 244 (1), 253-7

LISTAGE DE SEQUENCES

<110> DIATOS

<120> COMPOSITION ANTI-BACTERIENNE, PLUS PARTICULIEREMENT CONTRE LES BACTERIES GRAM NEGATIF, COMPRENANT UN PEPTIDE ET UN AGENT ANTI-BACTERIEN AVANTAGEUSEMENT HYDROPHOBE

<130> 33356/PCT

<140> PCT/FR04/xxx
<141> 2004-08-13

<150> FR03/09962 et EP 03292030.8
<151> 2003-08-14

<160> 23

<170> PatentIn version 3.1

<210> 1
<211> 16
<212> PRT
<213> Homo sapiens
<400> 1

```
Arg Lys Lys Arg Arg Arg Glu Ser Arg Lys Lys Arg Arg Arg Glu Ser
1               5               10              15
```

<210> 2
<211> 18
<212> PRT
<213> Homo sapiens
<400> 2

```
Arg Lys Lys Arg Arg Arg Glu Ser Arg Arg Ala Arg Arg Ser Pro Arg
1               5               10              15

His Leu
```

<210> 3
<211> 17
<212> PRT
<213> Homo sapiens
<400> 3

```
Gly Arg Pro Arg Glu Ser Gly Lys Lys Arg Lys Arg Lys Arg Leu Lys
1               5               10              15

Pro
```

<210> 4
<211> 15
<212> PRT
<213> Homo sapiens
<400> 4

```
Gly Lys Arg Lys Lys Lys Gly Lys Leu Gly Lys Lys Arg Asp Pro
```

1          5          10          15

<210> 5
<211> 17
<212> PRT
<213> Homo sapiens
<400> 5

```
Gly Lys Arg Lys Lys Lys Gly Lys Leu Gly Lys Lys Arg Pro Arg Ser
1                   5                   10                  15

Arg
```

<210> 6
<211> 16
<212> PRT
<213> Homo sapiens
<400> 6

```
Leu Arg Arg Glu Arg Gln Ser Arg Leu Arg Arg Glu Arg Gln Ser Arg
1                   5                   10                  15
```

<210> 7
<211> 22
<212> PRT
<213> Homo sapiens
<400> 7

```
Gly Ala Tyr Asp Leu Arg Arg Arg Glu Arg Gln Ser Arg Leu Arg Arg
1                   5                   10                  15

Arg Glu Arg Gln Ser Arg
            20
```

<210> 8
<211> 19
<212> PRT
<213> Homo sapiens
<400> 8

```
Val Lys Arg Gly Leu Lys Leu Arg His Val Arg Pro Arg Val Thr Arg
1                   5                   10                  15

Met Asp Val
```

<210> 9
<211> 10
<212> PRT
<213> Homo sapiens
<400> 9

```
Ala Lys Thr Gly Lys Arg Lys Arg Ser Gly
1               5               10
```

<210> 10
<211> 13
<212> PRT
<213> Homo sapiens
<400> 10

```
Gln Gly Lys Ser Lys Arg Glu Lys Lys Asp Arg Val Phe


    1               5                   10
```

<210> 11
<211> 18
<212> PRT
<213> Homo sapiens
<400> 11

```
Val Lys Arg Gly Leu Lys Leu Arg Gln Lys Tyr Asn Lys Arg Ala Met
1               5                   10                  15

Asp Tyr
```

<210> 12
<211> 22
<212> PRT
<213> Homo sapiens
<400> 12

```
Asn Pro Gly Val Ser Thr Val Val Leu Gly Ala Tyr Asp Leu Arg Arg
1               5                   10                  15

Arg Glu Arg Gln Ser Arg
                20
```

<210> 13
<211> 6
<212> PRT
<213> Homo sapiens
<400> 13

```
Arg Lys Lys Arg Arg Arg
1               5
```

<210> 14
<211> 3
<212> PRT
<213> Homo sapiens
<400> 14

```
        Arg Pro Arg
        1
```

<210> 15
<211> 7
<212> PRT
<213> Homo sapiens
<400> 15

```
        Lys Arg Lys Lys Lys Gly Lys
        1               5
```

<210> 16
<211> 4
<212> PRT
<213> Homo sapiens
<400> 16

```
        Arg Arg Glu Arg
        1
```

<210> 17
<211> 5
<212> PRT
<213> Homo sapiens
<400> 17

```
            Arg Arg Arg Glu Arg
            1               5
```

<210> 18
<211> 8
<212> PRT
<213> Homo sapiens
<400> 18

```
            Arg Arg Ala Arg Arg Ser Pro Arg
            1               5
```

<210> 19
<211> 9
<212> PRT
<213> Homo sapiens
<400> 19

```
            Lys Lys Arg Lys Arg Lys Arg Leu Lys
            1               5
```

<210> 20
<211> 3

<212> PRT
<213> Homo sapiens
<400> 20

```
                              Lys Lys Arg
                              1
```

<210> 21
<211> 7
<212> PRT
<213> Homo sapiens
<400> 21

```
                              Lys Lys Arg Pro Arg Ser Arg
                              1                   5
```

<210> 22
<211> 6
<212> PRT
<213> Homo sapiens
<400> 22

```
                              Arg Leu Arg Arg Glu Arg
                              1                   5
```

<210> 23
<211> 7
<212> PRT
<213> Homo sapiens
<400> 23

```
                              Arg Leu Arg Arg Arg Glu Arg
                              1                   5
```